# EUROPEAN PATENT APPLICATION

(11) **EP 4 691 536 A1**
(43) Date of publication of application: **11.02.2026**
(21) Application number: 24778382.2
(22) Date of filing: 22.03.2024
(51) Int. Cl.: A61M 25/09, A61M 25/01

(54) **DRIVING ASSEMBLY OF GUIDEWIRE AND CATHETER**

(30) Priority: 30.03.2023 KR 20230042168
(71) Applicant: XCATH, Inc., Houston, Texas 77054 (US)
(72) Inventor: JANG, Hyung Jun, Suwon-si, Gyeonggi-do 16420 (KR); CHO, Joo Hyun, Siheung-si, Gyeonggi-do (KR)
(74) Representative: Patentanwälte Ruff, Wilhelm, Beier, Dauster & Partner mbB
(86) International application number: PCT/IB2024/052746
(87) International publication number: WO 2024/201230

(57) **Abstract**

A drive assembly according to one example of the present invention comprises a drive roller (11); first idler rollers (12) facing the drive roller (11); and a pinch frame (13) to which the first idler rollers (12) are coupled, wherein by movement of the pinch frame (13), the first idler rollers (12) may be moved toward the drive roller (11) or spaced apart from the drive roller (11), a guide wire (30) may be disposed between the first idler rollers (12) and the drive roller (11), the pinch frame (13) may comprise a biasing member (18) disposed in the movement direction of the pinch frame (13) to adjust the pressure applied to the guide wire (30), the guide wire (30) may be moved forward or rearward by driving of the drive roller (11), and when the guide wire (30) moves forward or rearward, the pinch frame (13) may be moved so that the first idler rollers (12) come into contact with the guide wire (30).

## Description

### [TECHNICAL FIELD]

The present invention relates to a drive assembly of a guide wire and a catheter used for minimally invasive interventional procedures.

### [BACKGROUND ART]

A guide wire is used to guide a secondary sheath (e.g., a catheter) along the guide wire to a desired location in the body, e.g., a mammalian body such as a human body. In one application, a guide wire is introduced into a body lumen, i.e., a blood vessel, through an incision through the patient's skin and lumen wall, and the introduced, or distal, end of the guide wire is guided therefrom to a desired location of the lumen, or in a desired location of a lumen which branches into, or from, the lumen into which the guide wire is introduced.

One problem with guide wire introduction systems is that it is difficult to have the distal end of the guide wire follow tortuous lumen geometry, or to guide the distal end into an intersecting lumen or branch lumen connected to the lumen within which the distal end of the guide wire is positioned. In some cases, a branch lumen the location of which is the intended destination of the distal end of the guide wire intersects a lumen the distal end of which is at a large angle, for example, greater than 45 degrees, and in some cases, greater than 90 degrees.

To facilitate the orientation control of the distal end of the guide wire, a robotic system is provided for reliably controlling movement of the guide wire. Also provided is a cassette to be used with the robotic system. By the cassette, a guide wire is guided to a desired location in the body by making forward, rearward, and rotational movements.

The drive assembly of the cassette comprises a drive roller and idler roller. The drive roller and idler roller come into contact with a guide wire during forward or rearward movement of the guide wire. At this time, a small contact surface between the guide wire and the drive roller and idler roller results in a problem of low friction between the guide wire and the drive roller and idler roller, leading to slippage during forward or rearward movement of the guide wire.

### [PRIOR ART DOCUMENT]

### [PATENT DOCUMENT]

(PATENT DOCUMENT 1) US 2017-0274181 A1

### [DESCRIPTION OF THE INVENTION]

### [TECHNICAL PROBLEM]

A technical problem to be solved by the present invention is to provide a drive assembly that reduces slippage of a guide wire.

### [TECHNICAL MEANS FOR SOLVING THE PROBLEM]

A drive assembly according to one example of the present invention comprises a drive roller (11); first idler rollers (12) facing the drive roller (11); and a pinch frame (13) to which the first idler rollers (12) are coupled, wherein by movement of the pinch frame (13), the first idler rollers (12) may be moved toward the drive roller (11) or spaced apart from the drive roller (11), a guide wire (30) may be disposed between the first idler rollers (12) and the drive roller (11), the pinch frame (13) may comprise a biasing member (18) disposed in the movement direction of the pinch frame (13) to adjust the pressure applied to the guide wire (30), the guide wire (30) may be moved forward or rearward by driving of the drive roller (11), and when the guide wire (30) moves to drive the guide wire forwardly or rearwardly, the pinch frame (13) may be moved so that the first idler rollers (12) come into contact with the guide wire (30).

In one embodiment, the pinch frame (13) may comprise a first frame member (13a) coupled to a first surface of the biasing member (18) and a second frame member (13b) coupled to a second surface of the biasing member (18) facing the first surface.

In one embodiment, the drive assembly may further comprise a guide member (19) disposed parallel to the biasing member (18) and coupled to the first and second frame members (13a, 13b).

In one embodiment, the drive assembly may further comprise a rack (14) coupled to the second frame member (13b) and a pinion (15) engaged with the rack (14), wherein the first idler rollers (12) may be moved toward the drive roller (11) or spaced apart from the drive roller (11) by driving of the pinion (15).

In one embodiment, the diameter of the drive roller (11) may be greater than the diameter of the first idler rollers (12).

In one embodiment, when the guide wire (30) moves forward or rearward, the point at which the guide wire (30) comes into contact with the drive roller (11) may be located at the center point of the points at which the guide wire (30) comes into contact with the first idler rollers (12).

In one embodiment, when the guide wire (30) moves forward or rearward, the guide wire (30) may form a main bend (M) that bends along a contact surface with the drive roller (11) and sub bends (S) that bend along a contact surface with the first idler rollers (12).

In one embodiment, the drive assembly may further comprise a sensor roller (11') comprising a sensor for detecting movement of the guide wire (30) when the guide wire (30) moves forward or rearward and second idler rollers (12'), wherein the second idler rollers (12') may be coupled to the pinch frame (13).

In one embodiment, by movement of the pinch frame (13), the second idler rollers (12') may be moved toward the sensor roller (11') or spaced apart from the sensor roller (11'), the guide wire (30) may be disposed between the second idler rollers (12') and the sensor roller (11'), the pinch frame (13) may comprise a biasing member (18) disposed in the movement direction of the pinch frame (13) to adjust the pressure applied to the guide wire (30), and when the guide wire (30) moves forward or rearward, the pinch frame (13) may be moved so that the second idler rollers (12') come into contact with the guide wire (30).

In one embodiment, when the guide wire (30) rotates, the first idler rollers (12) may be spaced apart from the drive roller (11) by movement of the pinch frame (13).

In one embodiment, when the guide wire (30) rotates, the second idler rollers (12') may be spaced apart from the sensor roller (11') by movement of the pinch frame (13).

In one example of the present invention, a catheter may be used instead of the guide wire (30).

### [EFFECT OF THE INVENTION]

By increasing the contact surfaces between the guide wire and the drive roller and the idler rollers, the frictional force between the guide wire and the drive roller and the idler rollers can be increased, and slippage of the guide wire can be reduced.

### [BRIEF DESCRIPTION OF THE DRAWINGS]

Fig. 1a is an exploded perspective view illustrating a cassette according to an example of the present invention.
Fig. 1b is an exploded plan view illustrating a cassette according to an example of the present invention.
Fig. 2a is a perspective view illustrating a drive assembly and a rotation assembly according to an example of the present invention.
Fig. 2b is a plan view illustrating a drive assembly and a rotation assembly according to an example of the present invention.
Fig. 2c is a front view illustrating a drive assembly and a rotation assembly according to an example of the present invention.
Fig. 2d is a rear view illustrating a drive assembly and a rotation assembly according to an example of the present invention.
Fig. 2e is a side view illustrating a drive assembly and a rotation assembly according to an example of the present invention.
Fig. 2f is a side view illustrating a drive assembly and a rotation assembly according to an example of the present invention.
Fig. 3 is a perspective view illustrating a drive assembly according to an example of the present invention.
Fig. 4a is a perspective view showing forward or rearward movement of a guide wire according to an example of the present invention.
Fig. 4b is a plan view showing forward or rearward movement of a guide wire according to an example of the present invention.
Fig. 4c is a side view showing forward or rearward movement of a guide wire according to an example of the present invention.
Fig. 4d is a side view showing forward or rearward movement of a guide wire according to an example of the present invention.
Fig. 5a is a perspective view showing rotational movement of a guide wire according to an example of the present invention.
Fig. 5b is a plan view showing rotational movement of a guide wire according to an example of the present invention.
Fig. 5c is a side view showing rotational movement of a guide wire according to an example of the present invention.
Fig. 5d is a side view showing rotational movement of a guide wire according to an example of the present invention.
Fig. 6a is a plan view showing bending of a guide wire according to an example of the present invention.
Fig. 6b is an enlarged partial plan view showing bending of a guide wire according to an example of the present invention.

### [BEST EMBODIMENTS TO CARRY OUT THE INVENTION]

Hereinafter, preferred examples of the present invention will be described with reference to the attached drawings.

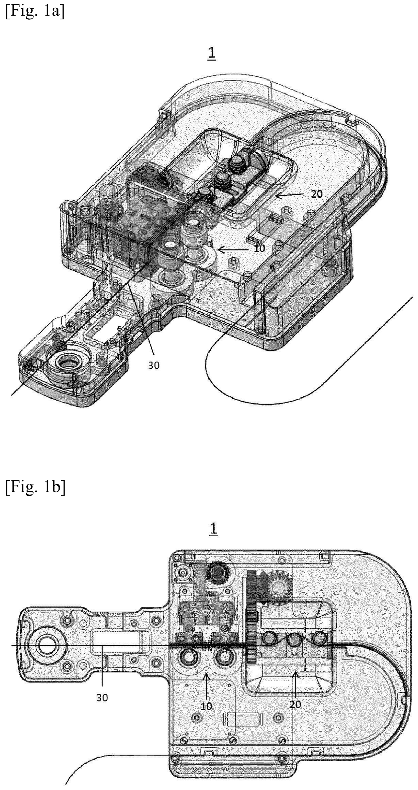

Figs. 1a and 1b respectively are an exploded perspective view and an exploded plan view of a cassette (1) according to an example of the present invention.

Referring to Figs. 1a and 1b, the cassette (1) may include a drive assembly (10) and a rotation assembly (20). A guide wire (30) may make forward, rearward, and rotational movements by the drive assembly (10) and the rotation assembly (20).

According to examples, the cassette (1) may be used for forward, rearward, and rotational movements of a catheter, and the structure of the rotation assembly (20) for using the guide wire (30) may be different from the structure of the rotation assembly (20) for using the catheter.

A description of the features included in the drive assembly (10) and the rotation assembly (20) will be provided below with reference to the drawings.

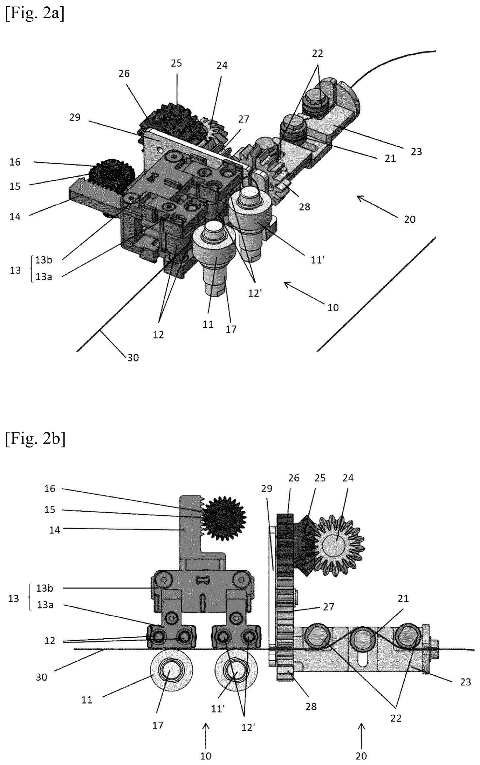

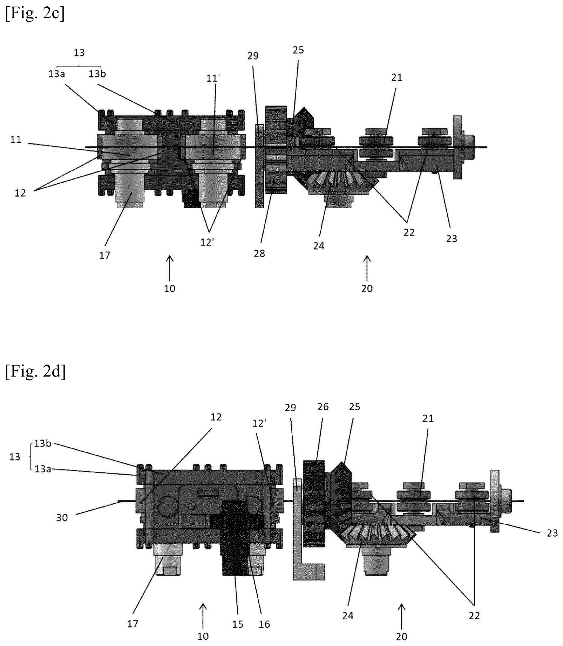

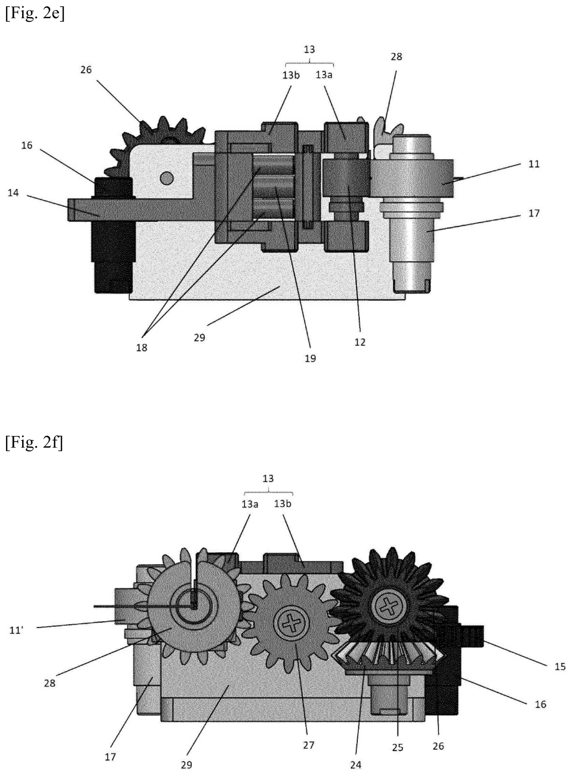

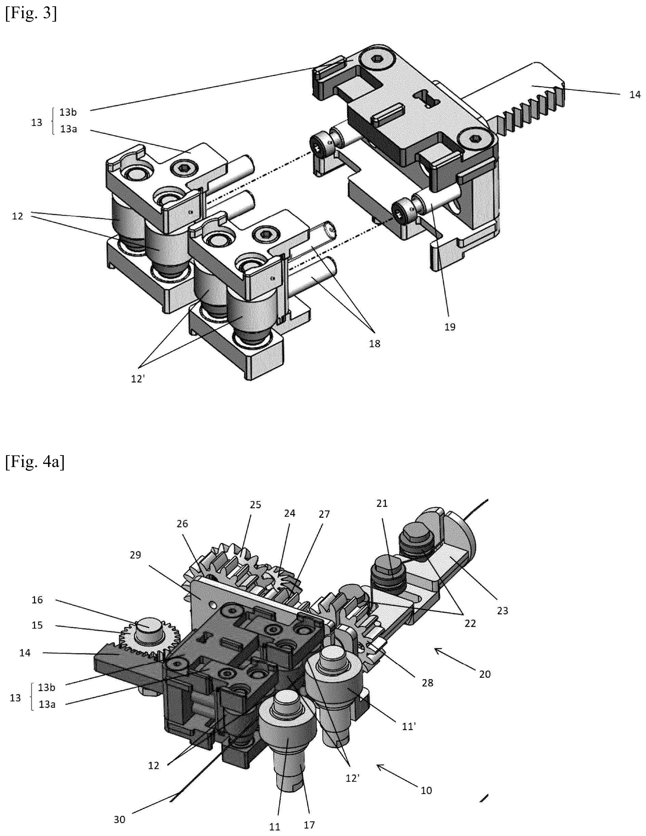

Figs. 2a to 2f respectively are a perspective view, a plan view, a front view, a rear view, a side view, and a side view illustrating a drive assembly and a rotation assembly according to an example of the present invention. Fig. 3 is a perspective view illustrating a drive assembly according to an example of the present invention.

The drive assembly (10) may comprise a drive roller (11), a sensor roller (11'), first idler rollers (12), second idler rollers (12'), a pinch frame (13), a rack (14), a pinion (15), a pinch shaft (16), a drive shaft (17), a biasing member (18), and a guide member (19).

The the drive roller (11) and the first idler rollers (12) face each other, and the guide wire (30) comes into contact with the the drive roller (11) and the first idler rollers (12) between the drive roller (11) and the first idler rollers (12).

If the contact surfaces between the guide wire (30) on the one hand and the rollers on the other hand are small, there may be a problem of slippage of the guide wire (30) due to low frictional force between the guide wire (30) and the rollers. The present invention proposes a structure for solving this problem.

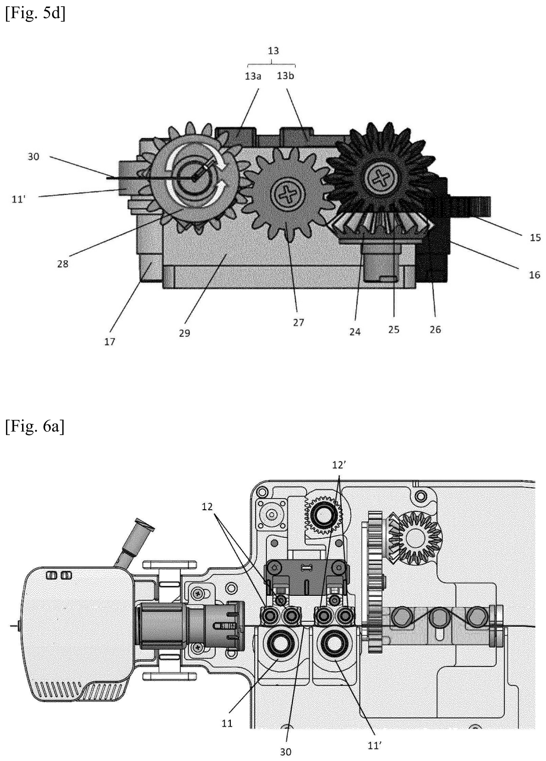

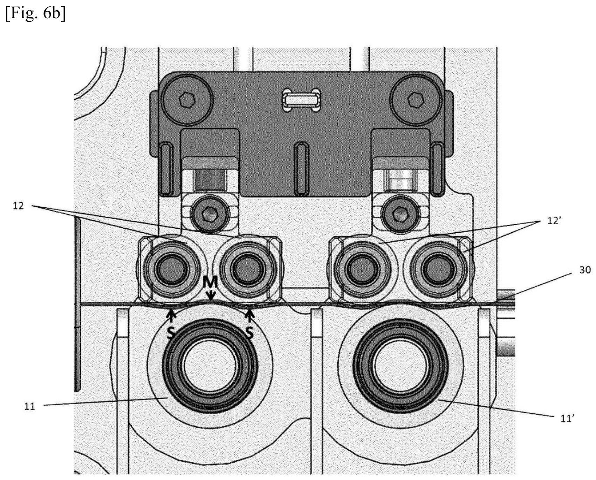

Referring to Figs. 6a and 6b, when the guide wire (30) moves forward or rearward, the guide wire (30) forms a main bend (M) that bends along a contact surface with the drive roller (11) and sub bends (S) that bend along a contact surface with the first idler rollers (12).

The increased contact surfaces between the guide wire (30) and the drive roller (11) and the first idler rollers (12), increases the frictional force between the guide wire (30) and the drive roller (11) and the first idler rollers (12), and reduce slippage of the guide wire (30).

Referring to Figs. 2a to 2f and 3 again, the diameter of the drive roller (11) may be greater than the diameter of the first idler rollers (12). When the guide wire (30) is moved forward or rearward, the locations at which the guide wire (30) comes into contact with the drive roller (11) will be located at the center point of the points at which the guide wire (30) comes into contact with the first idler rollers (12).

The sensor roller (11') may comprise a sensor for detecting movement of the guide wire (30) when the guide wire (30) moves forward or rearward.

The pinch frame (13) may comprise a first frame member (13a) and a second frame member (13b). The first frame member (13a) and the second frame member (13b) may be coupled to each other. The first idler rollers (12), the second idler rollers (12'), the biasing member (18), and the guide member (19) may be coupled to the first frame member (13a). The first idler rollers (12), the rack (14), the biasing member (18), and the guide member (19) may be coupled to the second frame member (13b). By movement of the pinch frame (13), the first idler rollers (12) may be moved toward the drive roller (11), or be spaced apart from the drive roller (11). By movement of the pinch frame (13), the second idler rollers (12') may be moved toward the sensor roller (11') or be spaced apart from the sensor roller (11'). When the guide wire (30) is moved forward or rearward, the pinch frame (13) is moved so that the first idler rollers (12) come into contact with the guide wire (30), and the guide wire (30) may be moved forward or rearward by driving of the drive roller (11). In addition, when the guide wire (30) moves forward or rearward, the pinch frame (13) may be moved so that the second idler rollers (12') come into contact with the guide wire (30).

The rack (14) is coupled to the second frame member (13b) and it is engaged with the pinion (15). The rack (14) may extend in a direction perpendicular to the coupling surface with the second frame member (13b). As the pinion (15) rotates, the rack (14) is moved forward or rearward , which causes the first idler rollers (12) to move forward or rearward .

The pinion (15) is engaged with the rack (14) and surrounds the pinch shaft (16). The pinion (15) may be separate from or integral with the pinch shaft (16).

The pinch shaft (16) is surrounded by the pinion (15) and is rotated by to a separate feature not shown. As the pinch shaft (16) rotates, the pinion (15) rotates.

The drive shaft (17) is surrounded by the drive roller (11), and it is rotated by a separate feature not shown. As the drive shaft (17) rotates, the drive roller (11) is rotated.

The biasing member (18) is disposed between the first frame member (13a) and the second frame member (13b) and coupled to the first frame member (13a) and the second frame member (13b). The biasing member (18) may be disposed in the moving direction of the pinch frame (13) to adjust the pressure applied to the guide wire (30), which is disposed between the first idler rollers (12) and the drive roller (11) and between the second idler rollers (12') and the sensor roller (11'). Specifically, as the rack (14) moves forward in the direction toward the first idler rollers (12), the first idler rollers (12) may move forward in the direction toward the guide wire (30), so that the first idler rollers (12) may pinch the guide wire (30) together with the drive roller (11). At this time, due to the elasticity of the biasing member (18), the rack (14) may move forward more strongly in the direction toward the first idler rollers (12), whereby the first idler rollers (12) may pinch more strongly the guide wire (30) together with the drive roller (11).

The biasing member (18) may be a feature to allow the first idler rollers (12) and the drive roller (11) to come into closer contact when the first idler rollers (12) are brought closer to the drive roller (11) by the pinch frame (13), and to allow the first idler rollers (12) and the drive roller (11) to be spaced further apart from each other when the first idler rollers (12) are moved away from the drive roller (11) by the pinch frame (13). The biasing member (18) may be a spring, for example, a coil spring or a leaf spring. According to examples, the biasing member (18) may be a material other than a spring, for example, a pneumatic cylinder.

The guide member (19) may be disposed parallel to the biasing member (18) between the first frame member (13a) and the second frame member (13b), so as to engage with the first frame member (13a) and the second frame member (13b). The guide member (19) may be a feature to prevent the first frame member (13a) and the first idler rollers (12) from being separated from the second frame member (13b).

The rotation assembly (20) comprises a center roller (21), side rollers (22), a rotary frame (23), a first bevel gear (24), a second bevel gear (25), a first spur gear (26), a second spur gear (27), a third spur gear (28), and a gear frame (29).

The center roller (21) is located between the side rollers (22). When the point at which the guide wire (30) comes into contact with the center roller (21) and the points at which the guide wire (30) comes into contact with the side rollers (22) are connected to each other, they may have a triangular shape, and the guide wire (30) may have an arch shape where a point that comes into contact with the center roller (21) protrudes. The rotational center axis of the guide wire (30) may have a shape of a straight line extending from the point at which the guide wire (30) comes into contact with the drive roller (11) to the points at which the guide wire (30) comes into contact with the side rollers (22). The point at which the guide wire (30) comes into contact with the center roller (21) may be a point at which the guide wire (30) is deviated from the rotational center axis.

The center roller (21) and the side rollers (22) are coupled to the rotary frame (23). The rotary frame (23) is coupled to the third spur gear (28), so that as the third spur gear (28) rotates, the rotary frame (23) also rotates, and the center roller (21), the side rollers (22), and the guide wire (30) may rotate together.

The first bevel gear (24) is rotated by a separate feature not shown. As the first bevel gear (24) rotates, the second bevel gear (25), the first spur gear (26), the second spur gear (27), and the third spur gear (28) sequentially rotate, thereby ultimately allowing the guide wire (30) to rotate.

The first spur gear (26), the second spur gear (27), and the third spur gear (28) may be coupled to the gear frame (29), and the drive assembly (10) and the rotation assembly (20) may be distinguished by the gear frame (29).

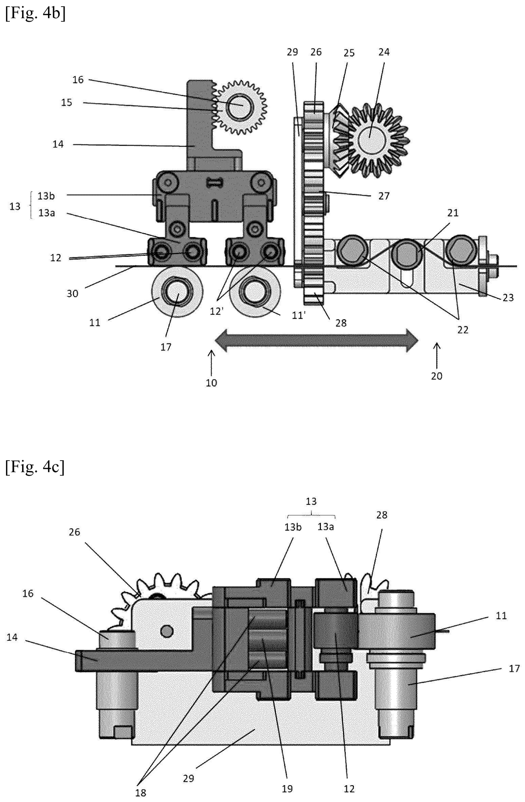

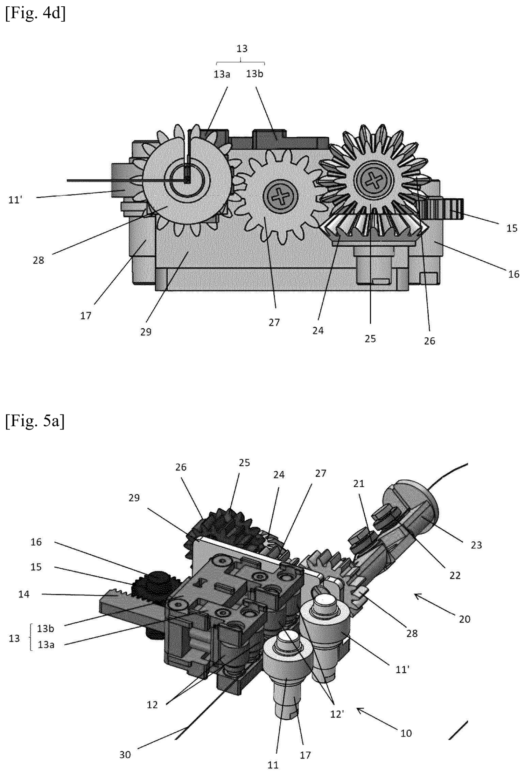

Figs. 4a to 4d respectively are a perspective views, a plan view, a side view, and a side view showing forward or rearward movement of a guide wire (30) according to an example of the present invention.

Referring to Figs. 4a to 4d, the forward or rearward movement of the guide wire (30) can be understood.

First, for forward movement of the guide wire (30), the guide wire (30) may be pinched. This is a state in which the guide wire (30) comes into contact with both the drive roller (11) and the first idler rollers (12). Whether the guide wire (30) is pinched or not may be determined by forward movement of the first idler rollers (12). As the pinion (15) rotates, the rack (14) may move forward, which causes forward movement of the first idler rollers (12). That is, the first idler rollers (12) may be moved toward the drive roller (11) or spaced apart from the drive roller (11) by driving of the pinion (15). This may mean that the first idler rollers (12) can move forward without a gear directly coupled to the first idler rollers (12).

In order to move the guide wire (30) forward, the drive shaft (17) may rotate first. As the drive shaft (17) rotates, the drive roller (11) rotates, whereby rotational movement of the first idler rollers (12) may be induced. The guide wire (30) may move forward due to rotational movement of the drive roller (11) and the first idler rollers (12).

At this time, by using the first idler rollers (12), which are a plurality of rollers facing the drive roller (11), the contact surfaces between the guide wire (30) and the drive roller (11) and the first idler rollers (12) may be increased, and the frictional force between the guide wire (30) and the drive roller (11) and the first idler rollers (12) may be increased, and slippage of the guide wire (30) with respect to the contacting surfaces of the drive roller (11) and the first idler rollers (12) may be reduced.

For reference, rearward movement of the guide wire (30) is different from forward movement of the guide wire (30) in the direction only, and the matters regarding the increased frictional force during forward movement of the guide wire (30) can be equally applied to the rearward movement of the guide wire (30).

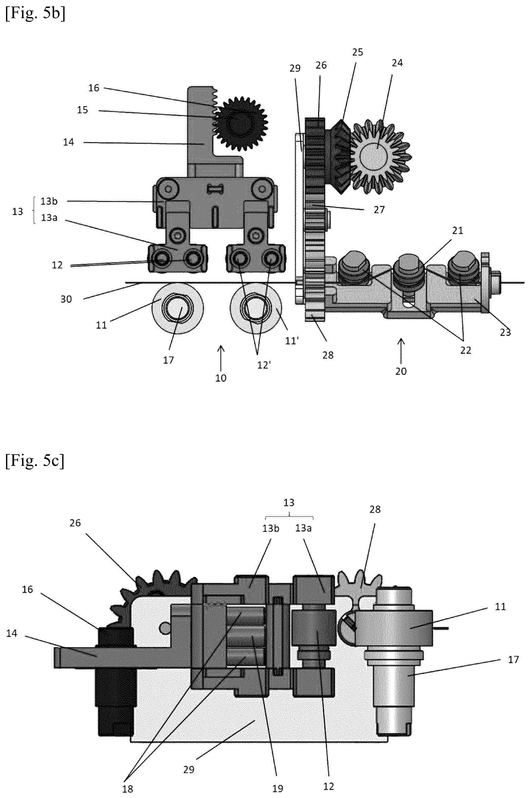

Figs. 5a to 5d respectively are a perspective view, a plan view, a side view, and a side view showing rotational movement of a guide wire (30) according to an example of the present invention.

Referring to Figs. 5a to 5d, the rotational movement of the guide wire (30) can be understood.

First, for rotational movement of the guide wire (30), the guide wire (30) is not pinched. This may mean that the guide wire (30) is not in a state in which it is in contact with any of the drive roller (11) and the first idler rollers (12).

When the guide wire (30) rotates, by movement of the pinch frame (13), the first idler rollers (12) are spaced from the drive roller (11) and the second idler rollers (12') are spaced apart from the sensor roller (11').

In order to rotate the guide wire (30), the first bevel gear (24) is rotated first. As the first bevel gear (24) rotates, the second bevel gear (25), the first spur gear (26), the second spur gear (27), and the third spur gear (28) may sequentially rotate, thereby ultimately causing the guide wire (30) to rotate.

Meanwhile, the drive assembly (10) may be used for forward movement or rotational movement of a catheter instead of being used for forward movement or rotational movement of the guide wire (30).

The present invention is not limited to the above-mentioned examples and the attached drawings, and various substitutions, modifications, and changes to the present invention may be made by those skilled in the art without departing from the technical spirit of the present invention as set forth in the claims, and such substitutions, modifications and changes should also be construed to be within the scope of the present invention.

### [DESCRIPTION OF REFERENCE NUMERALS]

1: cassette
10: drive assembly
11: drive roller
11': sensor roller
12: first idler rollers
12': second idler rollers
13: pinch frame
13a: first frame member
13b: second frame member
14: rack
15: pinion
16: pinch shaft
17: drive shaft
18: biasing member
19: guide member
20: rotation assembly
21: center roller
22: side roller
23: rotary frame
24: first bevel gear
25: second bevel gear
26: first spur gear
27: second spur gear
28: third spur gear
29: gear frame
30: guide wire
M: main bend
S: sub bend

## Claims

1. A drive assembly (10) comprising:
a drive roller (11);
first idler rollers (12) facing the drive roller (11); and
a pinch frame (13) to which the first idler rollers (12) are coupled,
wherein by movement of the pinch frame (13), the first idler rollers (12) are moved toward the drive roller (11) or spaced apart from the drive roller (11),
a guide wire (30) is disposed between the first idler rollers (12) and the drive roller (11),
the pinch frame (13) comprises a biasing member (18) disposed in the movement direction of the pinch frame (13) to adjust the pressure applied to the guide wire (30),
the guide wire (30) is moved forward or rearward by driving of the drive roller (11), and when the guide wire (30) moves forward or rearward, the pinch frame (13) is moved so that the first idler rollers (12) come into contact with the guide wire (30).

2. The drive assembly (10) according to claim 1,
wherein the pinch frame (13) comprises a first frame member (13a) coupled to a first surface of the biasing member (18) and a second frame member (13b) coupled to a second surface of the biasing member (18) facing the first surface.

3. The drive assembly (10) according to claim 2,
wherein the drive assembly (10) further comprises a guide member (19) disposed parallel to the biasing member (18) and coupled to the first and second frame members (13a, 13b).

4. The drive assembly (10) according to claim 2,
wherein the drive assembly (10) further comprises a rack (14) coupled to the second frame member (13b) and a pinion (15) engaged with the rack (14), wherein the first idler rollers (12) are moved toward the drive roller (11) or spaced apart from the drive roller (11) by driving of the pinion (15).

5. The drive assembly (10) according to claim 1,
wherein the diameter of the drive roller (11) is greater than the diameter of the first idler rollers (12).

6. The drive assembly (10) according to claim 1,
wherein when the guide wire (30) moves forward or rearward, the point at which the guide wire (30) comes into contact with the drive roller (11) is located at the center point of the points at which the guide wire (30) comes into contact with the first idler rollers (12).

7. The drive assembly (10) according to claim 1,
wherein when the guide wire (30) moves forward or rearward, the guide wire (30) forms a main bend (M) that bends along a contact surface with the drive roller (11) and sub bends (S) that bend along a contact surface with the first idler rollers (12).

8. The drive assembly (10) according to claim 1,
wherein the drive assembly (10) further comprises a sensor roller (11') comprising a sensor for detecting movement of the guide wire (30) when the guide wire (30) moves forward or rearward and second idler rollers (12'), wherein the second idler rollers (12') are coupled to the pinch frame (13).

9. The drive assembly (10) according to claim 8,
wherein by movement of the pinch frame (13), the second idler rollers (12') are moved toward the sensor roller (11') or spaced apart from the sensor roller (11'),
the guide wire (30) is disposed between the second idler rollers (12') and the sensor roller (11'),
the pinch frame (13) comprises a biasing member (18) disposed in the movement direction of the pinch frame (13) to adjust the pressure applied to the guide wire (30), and
when the guide wire (30) moves forward or rearward, the pinch frame (13) is moved so that the second idler rollers (12') come into contact with the guide wire (30).

10. The drive assembly (10) according to claim 1,
wherein when the guide wire (30) rotates, the first idler rollers (12) are spaced apart from the drive roller (11) by movement of the pinch frame (13).

11. The drive assembly (10) according to claim 10,
wherein when the guide wire (30) rotates, the second idler rollers (12') are spaced apart from the sensor roller (11') by movement of the pinch frame (13).

12. The drive assembly (10) according to any one of claims 1 to 11,
wherein a catheter is used instead of the guide wire (30).
